# FASCICULE DE BREVET EUROPEEN

(11) **EP 1 248 652 B1**
(45) Date de publication et mention de la délivrance du brevet: **06.04.2005**
(21) Numéro de dépôt: 01903860.3
(22) Date de dépôt: 03.01.2001
(51) Int. Cl.: A61K 47/02, A61K 47/12, A61K 31/4965

(54) **SOLUTION AQUEUSE STABLE DE DEOXYFRUCTOSAZINE**
STABILE WÄSSRIGE LÖSUNG VON DEOXYFRUCTOSAZINE
STABLE AQUEOUS DEOXYFRUCTOSAZINE SOLUTION

(30) Priorité: 06.01.2000 FR 0000138
(43) Date de publication de la demande: 16.10.2002
(73) Titulaire: Aventis Pharma S.A., 92160 Antony (FR)
(72) Inventeur: BASTIN, Richard, South Okendon, Essex RM15 4BE (GB); HART, Malcolm, Charles, Leigh-on-Sea, Essex SS9 3RL (GB); HUGHES, Nicholas, Romford, Essex RM1 4QR (GB)
(74) Mandataire: Rousseau, Pierrick Edouard
(86) Numéro de dépôt international: PCT/FR2001/000015
(87) Numéro de publication internationale: WO 2001/049261

(56) Documents cités:
- WO-A-97/28813
- HIRONOBU TSUCHIDA ET AL: "FORMATION OF DEOXY-FRUCTOSAZINE AND ITS 6-ISOMER ON THE BROWNING REACTION BETWEEN GLUCOSE AND AMMONIA IN WEAK ACIDIC MEDIUM" AGRICULTURAL AND BIOLOGICAL CHEMISTRY,JP,JAPAN SOCIETY FOR BIOSCIENCE, BIOTECHNOLOGY AND AGROCHEMISTRY,, vol. 37, no. 11, 1 novembre 1973 (1973-11-01), pages 2571-2578, XP000614905 ISSN: 0002-1369

## Description

La présente invention concerne une solution aqueuse stable de déoxyfructosazine notamment pour son utilisation pharmaceutique pour administration orale.

La déoxyfructosazine ou (2-[(1R,2S,3R)(1,2,3,4-tétrahydroxybutyl]-5-[2'S,3'R)(2',3',4'-trihydroxybutylpyrazine)] est connue pour ses propriétés antidiabétiques (WO97/28813).

Les solutions aqueuses et notamment celles pour administration orale doivent être physicochimiquement stables pendant plusieurs semaines ou mois et ce, dans des conditions de température variable.

Il a maintenant été trouvé que pour avoir un maximum de stabilité, les solutions aqueuses de déoxyfructosazine doivent présenter un pH de 3 à 5 et, de préférence de 3,5 à 4,5 et, plus particulièrement de 4.

En dehors de cette fourchette de pH, l'instabilité de la solution s'accroit rapidement et la solution contient des produits de dégradation en quantité incompatible avec un usage pharmacologique. Ainsi, le pourcentage d'un produit de dégradation A devient élevé et inacceptable lorsque le pH de la solution aqueuse diminue et le pourcentage d'un produit de dégradation B devient élevé et inacceptable lorsque le pH de la solution aqueuse augmente.

Le pH de la solution aqueuse est obtenu au moyen d'un acide pharmaceutiquement acceptable ou d'un système tampon pharmaceutiquement acceptable comprenant un acide et une base. Comme acide pharmaceutiquement acceptable, on peut utiliser par exemple l'acide citrique, l'acide phosphorique, l'acide acétique et l'acide tartrique. Comme base du système tampon, on peut utiliser, notamment, le citrate trisodique, le phosphate disodique, le tartrate disodique, l'acétate de sodium et la soude.

De préférence, la solution aqueuse de déoxyfructosazine est amenée au pH de 3 à 5 et, de préférence de 3,5 à 4,5 et, plus particulièrement, de 4 au moyen d'un système tampon. Comme système tampon préféré on peut citer les systèmes acide citrique et citrate trisodique ou acide phosphorique et soude.

La quantité de déoxyfructosazine dans la solution orale est de 1 à 150 mg/ml et, en particulier de 10 à 100 mg/ml.

L'étude de stabilité des solutions aqueuses de déoxyfructosazine a été effectuée selon le protocole suivant :

Une solution de déoxyfructosazine (1 mg/ml) est préparée à partir d'une solution aqueuse d'acide phosphorique 0,05M. Des échantillons de la solution sont ajustées à pH 2, 3, 4, 5, 6, 7, 8 et 9 par addition de soude. Les solutions ainsi obtenues sont conservées dans des flacons de verre protégés de la lumière à 4°C (solution témoin), 25°C, 45°C et 60°C. Après 2, 7 et 15 jours, les échantillons sont dilués 5 fois avec un tampon phosphate 20 mM, pH7 et la stabilité est examinée par chromatographie liquide haute pression (HPLC).

Les résultats après conservation des solutions pendant 15 jours sont les suivants :

| température de conservation | pH de la solution | % de produit de décomposition calculé par rapport à l'aire totale des pics | |
|---|---|---|---|
| | | produit B | produit A |
| 45°C | 2 | ND | 1,52 |
| | 3 | ND | 0,16 |
| | 4 | ND | ND |
| | 5 | 0,03 | ND |
| | 6 | 0,21 | ND |
| | 7 | 0,69 | ND |
| | 8 | 0,95 | ND |
| | 9 | 1,92 | ND |
| 60°C | 2 | 0,02 | 6,94 |
| | 3 | 0,03 | 0,87 |
| | 4 | 0,05 | 0,07 |
| | 5 | 0,24 | ND |
| | 6 | 0,71 | ND |
| | 7 | 13,9 | ND |
| | 8 | 37,9 | ND |
| | 9 | 37,7 | ND |
| ND = non détecté | | | |

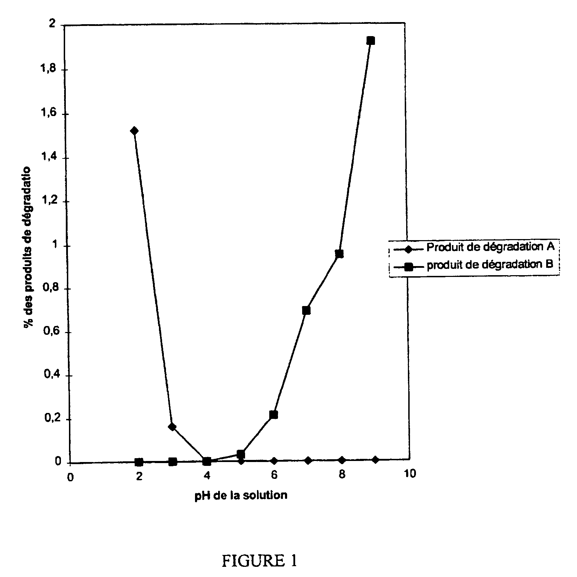

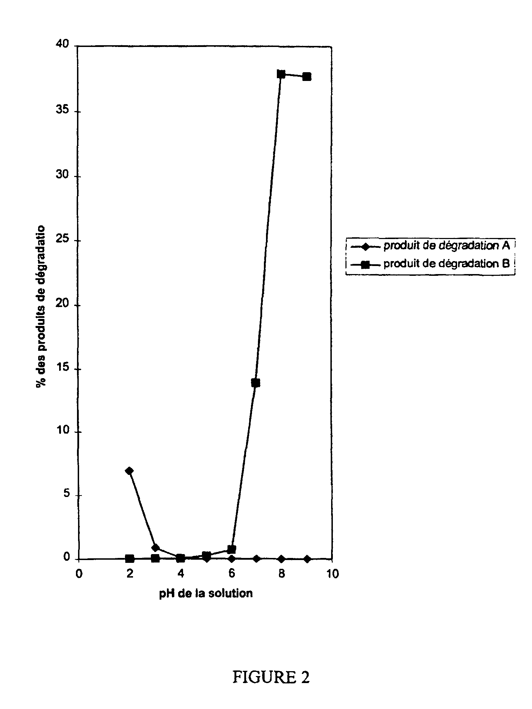

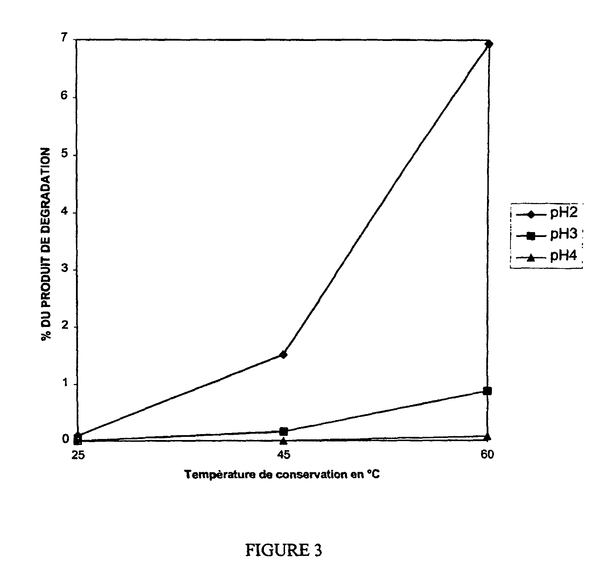

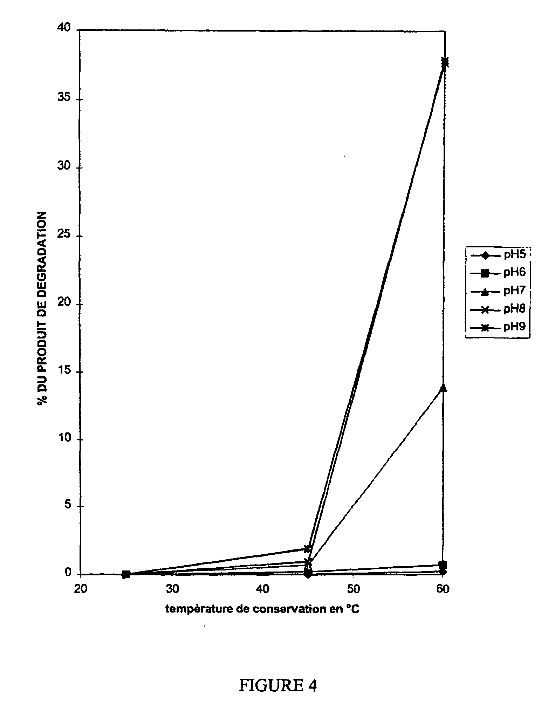

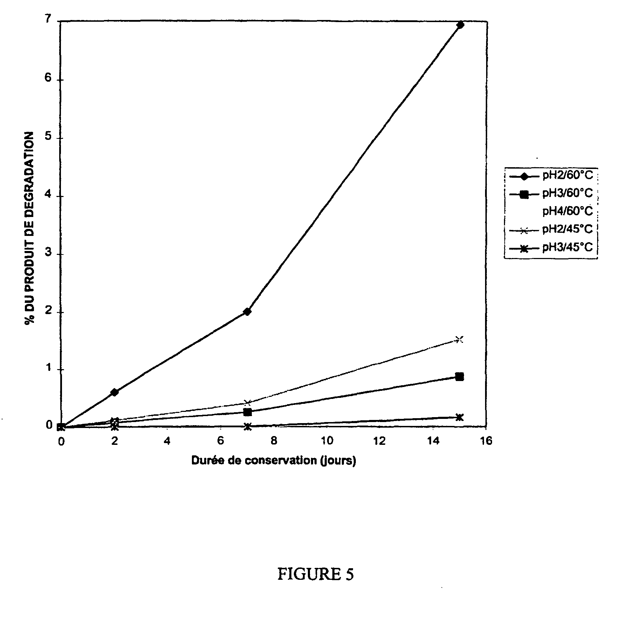

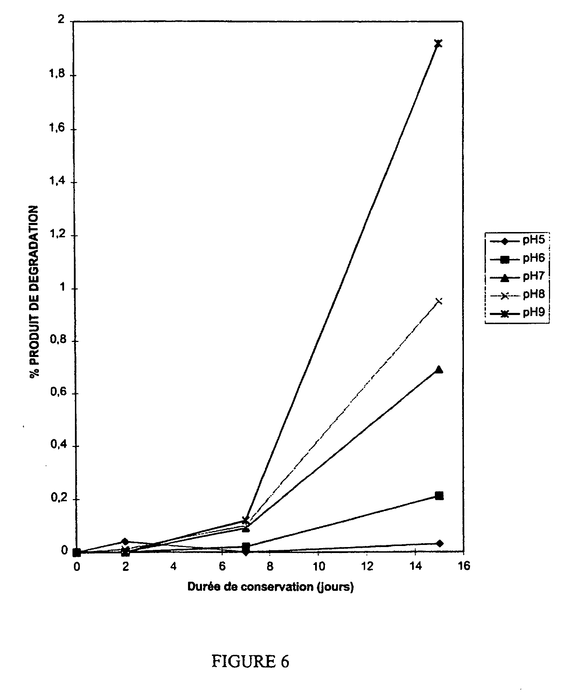

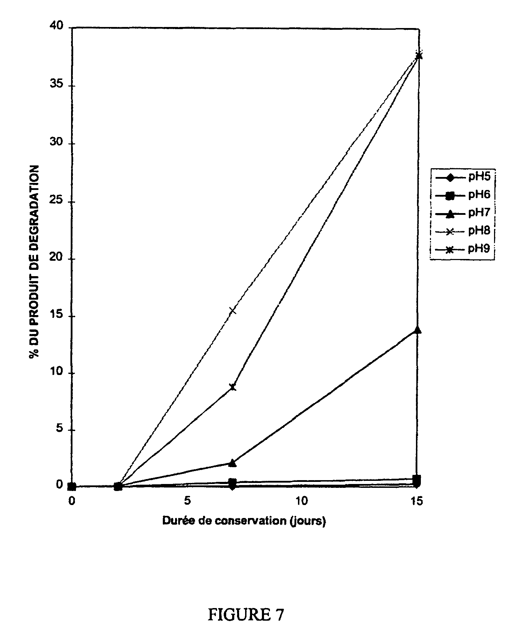

Les résultats des essais sont également résumés dans les figures 1 à 7.
La figure 1 montre les pourcentages des produits de dégradation A et B formés à différents pH après conservation des solutions aqueuses pendant 15 jours à 45°C.
La figure 2 montre les pourcentages des produits de dégradation A et B formés à différents pH après conservation des solutions aqueuses pendant 15 jours à 60°C.
La figure 3 montre la variation de formation du produit de dégradation A après conservation de solutions aqueuses à pH 2, 3 et 4 pendant 15 jours à 25°C, 45°C et 60°C.
La figure 4 montre la variation de formation du produit de dégradation B après conservation de solutions aqueuses à pH 2, 3 et 4 pendant 15 jours à 25°C, 45°C et 60°C.
La figure 5 montre la variation de formation du produit de dégradation A en fonction du temps de conservation des solutions aqueuses à pH 2 à 4 de solutions à 45 et 60°C.
La figure 6 montre la variation de formation du produit de dégradation B en fonction du temps de conservation des solutions aqueuses à pH 5 à 9 de solutions à 45°C.
La figure 7 montre la variation de formation du produit de dégradation B en fonction du temps de conservation des solutions aqueuses à pH 5 à 9 de solutions à 60°C.

Les solutions pour administration orale, peuvent contenir des tampons tels que ceux cités précédemment, des diluants, par exemple l'éthanol, le propylène glycol et la glycérine, des produits édulcorants comme la saccharine sodique, le cyclamate, des épaississants comme la saccharose (sucrose), les dérivés de cellulose, la Lycasin^{R} et le sorbitol, des aromatisants solubles dans l'eau, des préservateurs microbiens tels que le benzoate de sodium, l'acide benzoïque et des colorants. Elles peuvent éventuellement être stérilisées par toute méthode connue de l'homme de l'art et qui permet de ne pas dégrader la solution.

L'exemple suivant de solution orale aqueuse illustre l'invention :
100 mg de déoxyfructosazine
0,5 ml de propylèneglycol
70 mg d'acide citrique monohydrate
50 mg de citrate trisodique dihydrate
20 mg de benzoate de sodium
5 mg de saccharine sodium
2 µl d'aromatisant à l'ananas
0,8 mg de caramel
0,1 mg de riboflavine
eau déminéralisée en quantité suffisante pour 10 ml de solution

Les solutions selon l'invention peuvent être préparées par dissolution, sous agitation, à une température voisine de 20°C, de la déoxyfructosazine et des éventuels excipients dans de l'eau distillée et d'ajuster le pH au moyen d'un acide pharmaceutiquement acceptable. Dans le cas où l'on utilise un système tampon, on peut également dissoudre la déoxyfructosazine et les éventuels excipients dans de l'eau distillée contenant un acide pharmaceutiquement acceptable et ajuster le pH au moyen de la base du système tampon.

Ces solutions sont utiles pour la prévention et/ou le traitement du diabète de type 2 et ses complications.

## Revendications

1. Solution aqueuse de déoxyfructosazine présentant un pH de 3 à 5.

2. Solution selon la revendication 1 présentant un pH de 3,5 à 4,5.

3. Solution selon la revendication 1 présentant un pH de 4.

4. Solution selon l'une des revendications 1 à 3 contenant 1 à 150 mg/ml de déoxyfructosazine.

5. Solution selon l'une des revendication 1 à 3 contenant 10 à 100 mg/ml de déoxyfructosazine.

6. Solution selon l'une des revendications 1 à 5 pour laquelle le pH est ajusté au moyen d'un acide pharmaceutiquement acceptable.

7. Solution selon la revendication 6 pour laquelle l'acide pharmaceutiquement acceptable est choisi parmi l'acide citrique, l'acide phosphorique, l'acide tartrique et l'acide acétique.

8. Solution selon l'une des revendications 1 à 5 pour laquelle le pH est ajusté au moyen d'un système tampon pharmaceutiquement acceptable.

9. Solution selon la revendication 8 pour laquelle le système tampon est composé d'un acide pharmaceutiquement acceptable et d'une base pharmaceutiquement acceptable.

10. Solution selon la revendication 9 pour laquelle l'acide du système tampon est choisi parmi l'acide citrique, l'acide phosphorique, l'acide tartrique et l'acide acétique.

11. Solution selon la revendication 9 pour laquelle la base du système tampon est choisi parmi le citrate trisodique, le phosphate disodique, le tartrate disodique et la soude.

12. Solution selon la revendication 8 pour laquelle le système tampon est composé d'acide citrique et de citrate de sodium.

13. Solution selon la revendication 8 pour laquelle le système tampon est composé d'acide phosphorique et de soude.

14. Solution selon l'une des revendications 1 à 13 pour administration orale.

15. Solution selon l'une des revendication 1 à 14 contenant un ou plusieurs diluents pharmaceutiquement acceptables.

16. Composition pharmaceutique contenant une solution selon l'une des revendication 1 à 15.

17. Composition pharmaceutique selon la revendication 16 pour administration orale.

18. Composition pharmaceutique selon la revendication 16 pour administration orale contenant 100 mg de déoxyfructosazine, 0,5 ml de propylèneglycol, 70 mg d'acide citrique monohydrate, 50 mg de citrate trisodique dihydrate, 20 mg de benzoate de sodium, 5 mg de saccharine sodium, 2 µl d'aromatisant à l'ananas, 0,8 mg de caramel, 0,1 mg de riboflavine, eau déminéralisée en quantité suffisante pour 10 ml de solution.

## Patentansprüche

1. Wässrige Desoxyfructosazin-Lösung, die einen pH von 3 bis 5 aufweist.

2. Lösung gemäß Anspruch 1, die einen pH von 3,5 bis 4,5 aufweist.

3. Lösung gemäß Anspruch 1, die einen pH von 4 aufweist.

4. Lösung gemäß einem der Ansprüche 1 bis 3, die 1 bis 150 mg/ml Desoxyfructosazin enthält.

5. Lösung gemäß einem der Ansprüche 1 bis 3, die 10 bis 100 mg/ml Desoxyfructosazin enthält.

6. Lösung gemäß einem der Ansprüche 1 bis 5, für die der pH mittels einer pharmazeutisch unbedenklichen Säure eingestellt wird.

7. Lösung gemäß Anspruch 6, für die die pharmazeutisch unbedenkliche Säure ausgewählt ist unter Citronensäure, Phosphorsäure, Weinsäure und Essigsäure.

8. Lösung gemäß einem der Ansprüche 1 bis 5, für die der pH mittels eines pharmazeutisch unbedenklichen Puffersystems eingestellt wird.

9. Lösung gemäß Anspruch 8, für die das Puffersystem aus einer pharmazeutisch unbedenklichen Säure und einer pharmazeutisch unbedenklichen Base besteht.

10. Lösung gemäß Anspruch 9, für die die Säure des Puffersystems ausgewählt ist unter Citronensäure, Phosphorsäure, Weinsäure und Essigsäure.

11. Lösung gemäß Anspruch 9, für die die Base des Puffersystems ausgewählt ist unter Trinatriumcitrat, Dinatriumphosphat, Dinatriumtartrat und Natriumhydroxid.

12. Lösung gemäß Anspruch 8, für die das Puffersystem aus Citronensäure und Natriumcitrat besteht.

13. Lösung gemäß Anspruch 8, für die das Puffersystem aus Phosphorsäure und Natriumhydroxid besteht.

14. Lösung gemäß einem der Ansprüche 1 bis 13 für eine orale Verabreichung.

15. Lösung gemäß einem der Ansprüche 1 bis 14, die ein oder mehrere pharmazeutisch unbedenkliche Verdünnungsmittel enthält.

16. Pharmazeutische Zusammensetzung, die eine Lösung gemäß einem der Ansprüche 1 bis 15 enthält.

17. Pharmazeutische Zusammensetzung gemäß Anspruch 16 für eine orale Verabreichung.

18. Pharmazeutische Zusammensetzung gemäß Anspruch 16 für eine orale Verabreichung, die 100 mg Desoxyfructosazin, 0,5 ml Propylenglykol, 70 mg Citronensäure Monohydrat, 50 mg Trinatriumcitrat Dihydrat, 20 mg Natriumbenzoat, 5 mg Saccharin-Natrium, 2 µl Ananas-Aroma, 0,8 mg Karamell, 0,1 mg Riboflavin, entmineralisiertes Wasser in einer für 10 ml Lösung ausreichenden Menge enthält.

## Claims

1. Aqueous deoxyfructosazine solution with a pH from 3 to 5.

2. Solution according to Claim 1, with a pH from 3.5 to 4.5.

3. Solution according to Claim 1, with a pH of 4.

4. Solution according to one of Claims 1 to 3, containing 1 to 150 mg/ml of deoxyfructosazine.

5. Solution according to one of Claims 1 to 3, containing 10 to 100 mg/ml of deoxyfructosazine.

6. Solution according to one of Claims 1 to 5, for which the pH is adjusted by means of a pharmaceutically acceptable acid.

7. Solution according to Claim 6, for which the pharmaceutically acceptable acid is chosen from citric acid, phosphoric acid, tartaric acid and acetic acid.

8. Solution according to one of Claims 1 to 5, for which the pH is adjusted by means of a pharmaceutically acceptable buffer system.

9. Solution according to Claim 8, for which the buffer system is composed of a pharmaceutically acceptable acid and a pharmaceutically acceptable base.

10. Solution according to Claim 9, for which the acid in the buffer system is chosen from citric acid, phosphoric acid, tartaric acid and acetic acid.

11. Solution according to Claim 9, for which the base in the buffer system is chosen from trisodium citrate, disodium phosphate, disodium tartrate and sodium hydroxide.

12. Solution according to Claim 8, for which the buffer system is composed of citric acid and sodium citrate.

13. Solution according to Claim 8, for which the buffer system is composed of phosphoric acid and sodium hydroxide.

14. Solution according to one of Claims 1 to 13, for oral administration.

15. Solution according to one of Claims 1 to 14, containing one or more pharmaceutically acceptable diluents.

16. Pharmaceutical composition containing a solution according to one of Claims 1 to 15.

17. Pharmaceutical composition according to Claim 16, for oral administration.

18. Pharmaceutical composition according to Claim 16, for oral administration, containing 100 mg of deoxyfructosazine, 0.5 ml of propylene glycol, 70 mg of citric acid monohydrate, 50 mg of trisodium citrate dihydrate, 20 mg of sodium benzoate, 5 mg of sodium saccharin, 2 µl of pineapple flavouring, 0.8 mg of caramel, 0.1 mg of riboflavine, and demineralized water in a quantity sufficient for 10 ml of solution.
